# EUROPÄISCHE PATENTANMELDUNG

(11) **EP 1 801 088 A1**
(43) Veröffentlichungstag der Anmeldung: **27.06.2007**
(21) Anmeldenummer: 05027725.0
(22) Anmeldetag: 19.12.2005
(51) Int. Cl.: C07C 17/156, C07C 19/045, B01J 23/72, B01J 8/18

(54) **Oxychlorierungsverfahren**

(71) Anmelder: VESTOLIT GmbH & Co. KG, 45772 Marl (DE)
(72) Erfinder: Kahsnitz, John, 45721 Haltern (DE); Steffens, Martin, 48147 Münster (DE); Träger, Michael, 45721 Haltern am See (DE); Weinmann, Dirk, 45657 Recklinghausen (DE)
(74) Vertreter: polypatent

(57) **Zusammenfassung**

Die vorliegenden Erfindung betrifft ein Verfahren zur Herstellung eines halogenierten Kohlenwasserstoffs in einem Wirbelschichtreaktor (1) umfassend die Schritte:
a) Einbringen von Sauerstoff, mindestens einem Olefin und mindestens einem Halogenwasserstoff in den Wirbelschichtreaktor, wobei der Wirbelschichtreaktor eine gasdurchlässige Bodenplatte (2) aufweist, oberhalb der Bodenplatte (2) ein Reaktionsraum gebildet wird in dem ein heterogener Katalysator angeordnet ist, der Reaktionsraum wenigstens einen Wärmetauscher (3) aufweist der einen solchen Abstand von der Bodenplatte aufweist, dass sich eine erste Reaktionszone (4) und eine zweite Reaktionszone (5) bilden, wobei die erste Reaktionszone (4) nicht durch den Wärmetauscher (3) temperiert wird, und
b) Entfernen des gebildeten halogenierten Kohlenwasserstoffs,
sowie einen Reaktor zur Durchführung des Verfahrens.

## Beschreibung

Die vorliegende Erfindung betrifft ein verbessertes Verfahren zur Oxychlorierung (OC) in Wirbelschichtreaktoren, sowie einen Wirbelschichtreaktor, in dem das vorgenannte Verfahren durchgeführt werden kann.

Die Grundlage der Oxychlorierung bildet der schon seit über 100 Jahren bekannte DEACON-Prozess, bei dem Chlor durch Oxidation von Chlorwasserstoff mit Sauerstoff über Kupfer-Katalysatoren erzeugt werden kann. Dabei sind jedoch nur unvollständige Umsätze möglich. Sind im Reaktionsgemisch aber Chlor-Akzeptoren, z. B. chlorierbare Kohlenwasserstoffe, vorhanden, die das entstehende Chlor jeweils sofort binden, so wird das Gleichgewicht ganz auf Seite der organischen Chlorverbindungen verschoben. Die Oxychlorierung ist als DEACON-Prozess "in situ" zu betrachten und lässt sich durch folgende Gleichung veranschaulichen:

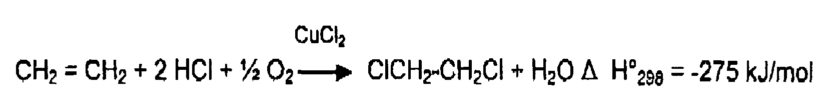

Selbstverständlich reagiert dabei der Sauerstoff nicht nur mit Chlorwasserstoff, sondern auch mit den Kohlenwasserstoffen zu Oxidationsprodukten.

Für die Umsetzung stehen bis zur technischen Reife entwickelte Verfahren, sowohl in der Gasphase an festen oder fluidisierten Kontakten, als auch in flüssiger Phase mit gelösten Katalysatoren zur Verfügung. Großtechnische Anwendung haben aber bis jetzt nur die Verfahren der Gasphase gefunden. Man benötigt für sie Temperaturen von 200 - 300 °C und Drücke von 3 - 10 bar; dagegen sind in der flüssigen Phase niedrigere Temperaturen von 170 - 190 °C, aber höhere Drücke von 15-20 bar erforderlich. Die extrem hohe Reaktionswärme macht den Reaktor zum kritischen Teil der Anlage. Die Umsätze sind bei allen Verfahren akzeptabel. Die Ausbeuten an Dichlorethan liegen je nach Verfahren zwischen 91 - 97 % bezogen sowohl auf Ethylen als auch auf Chlorwasserstoff. Etwa 2 - 3 % des Ethylens reagiert zu Kohlenoxiden.

Für die Oxychlorierung werden allgemein Kupferchloride als Katalysatoren verwendet.

Der Chemismus der Katalyse kann formelmäßig in folgende Teilschnitte zerlegt werden:

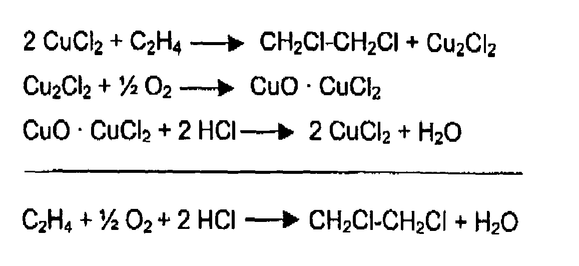

Als poröses Trägermaterial für den Katalysator finden fast ausschließlich aktiviertes Aluminiumoxid, Silicagel und Diatomeenerde Verwendung. Art und Herkunft des Trägers sowie Oberfläche und Porengrößenverteilung beeinflussen die Katalysatoreigenschaften bis zu einem bestimmten Grad.

Die großtechnisch angewandte Oxychlorierung in der Gasphase wird sowohl im Festbett (DE 2 440 864.8) als auch in der Wirbelschicht (DE 1 618 701) beschrieben.

Für die Oxychlorierung ist der Wirbelschichtreaktor gegenüber dem Festbettreaktor wegen des ausgezeichneten Wärmeübergangs besser geeignet. Die Gefahr der örtliche Überhitzung wird minimiert.

Ein wesentliches technisches Problem beim Wirbelschichtverfahren ist das Verflüchtigen der Kupferchloride unter den Reaktionsbedingungen bei Reaktionstemperaturen größer gleich 250°C. Außerdem stellen Werkstoffschädigungen des Reaktors über Korrosions- und Erosionsmechanismen ein Problem dar.

Ein Verfahren der Oxychlorierung wird in der DE 1 618 701 offenbart.

Ein wesentlicher Vorteil eines Wirbelschichtreaktors ist die deutlich höhere Leistungsdichte. So sind die für diesen Reaktortyp notwendigen Katalysatoren mit einem deutlich höheren Kupferanteil mit Gehalten am Gesamtkatalysator von bis zu ca. 12 Gew.% beaufschlagt.

Negativ ist jedoch die Zuverlässigkeit solcher Reaktoren. So kommt es häufig zu Ausfällen wegen Defekten an den Kühlrohren aufgrund von Korrosions/Erosionsschäden.

Eine Gegenmaßnahme gegen diesen korrosiven/erosiven Angriff ist die Ausstattung des Reaktors mit einem hochwertigen und resistenten Werkstoff. So wird der Nickelbasiswerkstoff Hastelloy C-276 als besonders resistent gegen die vorhandenen Reaktionsbedingungen angesehen. Wegen des sehr hohen finanziellen Aufwands wird dieser Weg ungern gewählt. Außerdem wird durch die veränderte Werkstoffauswahl das grundsätzliche verfahrenstechnische Problem nicht gelöst. Eine, wenn auch verlangsamte, Korrosion tritt weiterhin auf.

Der vorliegenden Erfindung liegt somit die technische Aufgabe zugrunde, ein verbessertes Verfahren zur Herstellung eines halogenierten Kohlenwasserstoffs bereitzustellen, bei dem die Effizienz und die Ausbeute an Produkt weiter verbessert wird, die Standzeiten des benötigten Katalysators erhöht sind, sowie die Verwendung von extrem hochwertigen Werkstoffen zur Herstellung eines Reaktors zur Durchführung der Oxychlorierung, vermieden wird.

Die technische Aufgabe der vorliegenden Erfindung wird gelöst durch ein Verfahren zur Herstellung eines halogenierten Kohlenwasserstoffs in einem Wirbelschichtreaktor 1 umfassend die Schritte:
a) Einbringen von Sauerstoff, mindestens einem Olefin und mindestens einem Halogenwasserstoff in den Wirbelschichtreaktor, wobei der Wirbelschichtreaktor eine gasdurchlässige Bodenplatte 2 aufweist, oberhalb der Bodenplatte 2 ein Reaktionsraum gebildet wird in dem ein heterogener Katalysator angeordnet ist, der Reaktionsraum wenigstens einen Wärmetauscher 3 aufweist der einen solchen Abstand von der Bodenplatte aufweist, dass sich eine erste Reaktionszone 4 und eine zweite Reaktionszone 5 bilden, wobei die erste Reaktionszone 4 nicht durch den Wärmetauscher 3 temperiert wird, und
b) Entfernen des gebildeten halogenierten Kohlenwasserstoffs.

Die Wirbelschichtreaktoren weisen üblicherweise eine gasdurchlässige Bodenplatte auf. Oberhalb dieser Bodenplatte wird der heterogene Katalysator angeordnet und der Eduktgasstrom wird durch die gasdurchlässige Bodenplatte zu dem Katalysator geleitet. Im Zusammenhang mit der vorliegenden Erfindung ist die erste Reaktionszone benachbart zur Bodenplatte. Die zweite Reaktionszone folgt dann der ersten Reaktionszone in Richtung des entstehenden Produktes. In Richtung des Produktstromes ist somit die räumliche Anordnung in dem Reaktor zunächst die gasdurchlässige Bodenplatte, dann die erste Reaktionszone, welche von der zweiten Reaktionszone gefolgt wird. Dem Reaktor können übliche weitere Aggregate nachgeschaltet sein, wie zum Beispiel Gaskühler oder Gaswäscher, die der Aufarbeitung des Produktstroms dienen.

In einer bevorzugten Ausführungsform ist der halogenierte Kohlenwasserstoff 1, 2 Dichlorethan, das Olefin ist Ethen und/oder der Halogenwasserstoff ist Chlorwasserstoff.

In dem Verfahren der vorliegenden Erfindung kann ein Wärmetauscher 3 verwendet werden, der dem Fachmann im Zusammenhang mit Oxychtorierungsverfahren bekannt ist. Vorzugsweise wird in dem Verfahren der vorliegenden Erfindung die zweite Reaktionszone 5 durch den Wärmetauscher 3 gekühlt. Der Wärmetauscher kann mit jedem geeigneten Kühlmittel betrieben werden.

Vorzugsweise ist in dem Verfahren der vorliegenden Erfindung der Abstand zwischen der Bodenplatte 2 und dem Wärmetauscher 3 mindestens 30 mm, weiter bevorzugt mindestens 40 mm, insbesondere mindestens 70 mm und am meisten bevorzugt mindestens 100 mm.

Vorzugsweise beträgt in dem Verfahren der vorliegenden Erfindung der Abstand zwischen der Bodenplatte 2 und dem Wärmetauscher 3 maximal 600 mm, weiter bevorzugt maximal 500 mm und am meisten bevorzugt maximal 400 mm.

In dem Verfahren der vorliegenden Erfindung kann der Wärmetauscher 3 wenigstens ein Kühlschlangenbündel umfassen. Alternativ ist es auch möglich, dass der Wärmetauscher mehrere Kühlschlangenbündel umfasst, die in einer Ebene innerhalb des Reaktors angeordnet sind.

In dem Verfahren der vorliegenden Erfindung kann als heterogener Katalysator jeder Katalysator verwendet werden, der für Oxychlorierungsverfahren geeignet ist. Insbesondere kann als heterogener Katalysator ein Katalysator verwendet werden, der als Wirkkomponente Kupfer enthält. Der heterogene Katalysator ist vorzugsweise teilchenförmig und weist weiter bevorzugt einen mittleren Korndurchmesser von 40 µm bis 80 µm auf.

Überraschenderweise ist die Korrosion des Reaktors durch das Verfahren der vorliegenden Erfindung vermindert. Es wird angenommen, ohne jedoch an diese Theorie gebunden zu sein, dass durch das Verfahren der vorliegenden Erfindung die Bildung von wässrigem Halogenwasserstoff verhindert wird und somit eine hierdurch bedingte Korrosion des Reaktors und insbesondere des Wärmetauschers vermieden wird. Dies führt zwangsläufig zu einer verlängerten Lebensdauer des Reaktors. Dieser Effekt ist aber überraschend, da die sehr stark exotherme Reaktion der Oxychlorierung im Regelfall eine intensive Kühlung des Reaktors benötigt und da die erste Reaktionszone durch den Wärmetauscher nicht temperiert wird, ist es somit erstaunlich, dass der Reaktorbereich, in dem sich die erste Reaktionszone befindet, nicht einer erhöhten thermischen Belastung, die zwangsläufig auch zu einem Austrag von aktiver Katalysatorkomponente führen würde, ausgesetzt ist.

Ein weiterer Gegenstand der vorliegenden Erfindung ist ein Wirbelschichtreaktor 1 zur Durchführung des Verfahrens. Der Wirbelschichtreaktor 1 zur Herstellung eines halogenierten Kohlenwasserstoffs umfasst mindestens einen Einlass für den Eduktstrom, mindestens einen Auslass für den Produktstrom, eine über dem Einlass angeordnete gasdurchlässige Bodenplatte 2 und einen oberhalb der Bodenplatte 2 gebildeten Reaktionsraum, wobei in dem Reaktionsraum wenigstens ein Wärmetauscher 3 angeordnet ist, der einen solchen Abstand von der Bodenplatte 2 aufweist, dass sich eine erste Reaktionszone 4 und eine zweite Reaktionszone 5 bildet, wobei die erste Reaktionszone 4 nicht durch den Wärmetauscher 3 temperiert wird.

Vorzugsweise ist der Wärmetauscher 3 in der zweiten Reaktionszone 5 so ausgelegt, dass er gekühlt werden kann.

In einer weiter bevorzugten Ausführungsform ist der Abstand zwischen der Bodenplatte 2 und dem Wärmetauscher 3 mindestens 30 mm, weiter bevorzugt mindestens 40 mm, insbesondere mindestens 70 mm und am meisten bevorzugt mindestens 100 mm.

Vorzugsweise beträgt der Abstand zwischen der Bodenplatte 2 und dem Wärmetauscher 3 maximal 600 mm, weiter bevorzugt beträgt der Abstand maximal 500 mm und am meisten bevorzugt maximal 400 mm. Der Wirbelschichtreaktor 3 hat vorzugsweise einen Durchmesser von 100 cm bis 10.000 cm, weiter bevorzugt von 200 cm bis 600 cm und am meisten bevorzugt von 300 cm bis 500 cm.

Der Wärmetauscher 3 des Wirbelschichtreaktors 1 umfasst vorzugsweise wenigstens ein Kühlschlangenbündel. In einer weiter bevorzugten Ausführungsform sind mehrere Kühlschlangenbündel in dem Wärmetauscher enthalten. Vorzugsweise ist in dem Reaktor wenigstens ein Zyklon enthalten, mit dem der Katalysator vom Produkt getrennt werden kann.

Die vorliegende Erfindung wird an weiteren bevorzugten Ausführungsformen erläutert.

Figur 1 ist eine schematische Darstellung eines bevorzugten Wirbelschichtreaktors der vorliegenden Erfindung.

In Figur 2 wird das Temperaturprofil innerhalb eines erfindungsgemäßen Wirbelschichtreaktors dargestellt.

Figur 3 ist eine schematische Darstellung eines bevorzugten Wirbelschichtreaktors des Standes der Technik.

In Figur 4 wird der Temperaturverlauf innerhalb eines Wirbelschichtreaktors des Standes der Technik dargestellt.

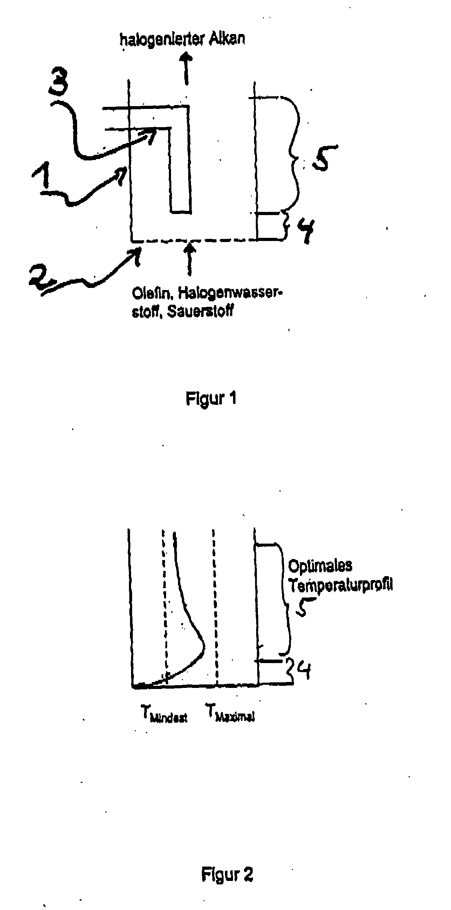

In Figur 1 wird ein Ausschnitt aus einem erfindungsgemäßen Wirbelschichtreaktor 1 dargestellt. Dargestellt ist die gasdurchlässige Bodenplatte 2, der zylinderförmige Mantel des Reaktors 1, sowie der Wärmetauscher 3. Die erste Reaktionszone 4 wird zwischen der Bodenplatte 2 und dem Wärmetauscher 3 gebildet. Die zweite Reaktionszone 5 wird im Bereich des Wärmetauschers 3 gebildet.

Wenn das Verfahren der vorliegenden Erfindung durchgeführt wird, stellt sich in dem Wirbelschichtreaktor vorzugsweise ein Temperaturprofil ein, welches in Figur 2 schematisch dargestellt ist. In der ersten Reaktionszone 4 stellt sich eine Mindesttemperatur ein, bei der die Selektivität und die Effizienz des Katalysators am Höchsten ist. Des Weiteren wird durch den Wärmetauscher 3 in der zweiten Reaktionszone 5 eine Temperatur erzielt, bei der eine Zersetzung des Katalysators vermindert wird. In dem Diagramm ist die entsprechende Mindesttemperatur mit T_{Mindest} dargestellt, sowie die maximale Temperatur mit T_{Maximal}. Die in der Figur 2 eingezeichnete Kurve stellt das Temperaturprofil innerhalb des Reaktors dar.

Durch die spezielle Konstruktion des Reaktors wird somit ein Temperaturprofil innerhalb des Reaktors erzielt, bei dem einerseits das Verfahren zur Herstellung von halogenierten Kohlenwasserstoffen besonders effizient durchgeführt werden kann, während zusätzlich eine Korrosion bzw. eine nachteilige Beeinflussung des Reaktors vermieden wird.

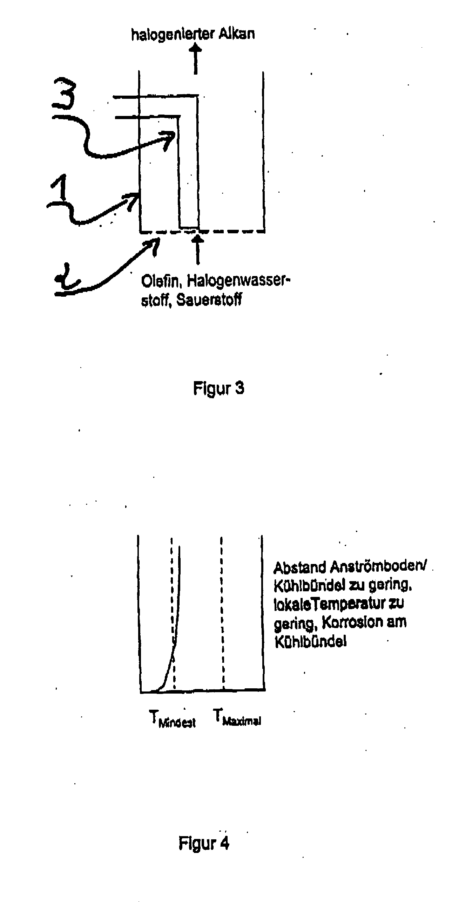

In Figur 3 wird ein Reaktor des Standes der Technik dargestellt. In dem in Figur 3 dargestellten Wirbelschichtreaktor 1 reicht der Wärmetauscher 3 bis dicht vor die Bodenplatte 2. Hieraus ergibt sich bei Durchführung des Verfahrens zur Herstellung eines halogenierten Kohlenwasserstoffs ein Temperaturprofil, welches in Figur 4 dargestellt ist. Aus der Figur 4 ist ersichtlich, dass der untere Bereich des Reaktors bei einer Temperatur gehalten wird, die zu niedrig ist, um ein effizientes Verfahren zur Herstellung von halogenierten Kohlenwasserstoffen durchzuführen. Aufgrund der zu niedrigen Temperatur wird einerseits die Effizienz des Verfahrens negativ beeinflusst und andererseits wird durch die zu niedrige Temperatur im unteren Bereich des Reaktors die Korrosion des Reaktors gefördert. Insbesondere findet aufgrund der zu geringen Temperatur im unteren Bereich des Reaktors eine Korrosion des Wärmetauschers statt.

Zusammenfassend kann festgestellt werden, dass durch das Verfahren der vorliegenden Erfindung, sowie den dazu geeigneten Reaktor die Korrosion des Reaktors wirksam verhindert wird. Zusätzlich wird die maximal zulässige Temperatur, bei der der Katalysator optimal arbeitet, nicht überschritten. Außerdem wird in dem Reaktor ein Temperaturprofil erzielt, welches dazu führt, dass in dem unteren Bereich des Wirbelschichtreaktors schneller eine optimale Temperatur zur Durchführung des Verfahrens zur Herstellung von halogenierten Kohlenwasserstoffen erreicht wird.

### Bezugszeichenliste

- 1: Reaktor (Ausschnitt)
- 2: Gasdurchlässige Bodenplatte
- 3: Wärmetauscher
- 4: Erste Reaktionszone
- 5: Zweite Reaktionszone

## Patentansprüche

1. Verfahren zur Herstellung eines halogenierten Kohlenwasserstoffs in einem Wirbelschichtreaktor (1) umfassend die Schritte:
a) Einbringen von Sauerstoff, mindestens einem Olefin und mindestens einem Halogenwasserstoff in den Wirbelschichtreaktor, wobei der Wirbelschichtreaktor eine gasdurchlässige Bodenplatte (2) aufweist, oberhalb der Bodenplatte (2) ein Reaktionsraum gebildet wird in dem ein heterogener Katalysator angeordnet ist, der Reaktionsraum wenigstens einen Wärmetauscher (3) aufweist der einen solchen Abstand von der Bodenplatte aufweist, dass sich eine erste Reaktionszone (4) und eine zweite Reaktionszone (5) bilden, wobei die erste Reaktionszone (4) nicht durch den Wärmetauscher (3) temperiert wird, und
b) Entfernen des gebildeten halogenierten Kohlenwasserstoffs.

2. Verfahren gemäß Anspruch 1, **dadurch gekennzeichnet, dass** der halogenierte Kohlenwasserstoff 1,2-Dichlorethan ist, das Olefin Ethen ist und/oder der Halogenwasserstoff Chlorwasserstoff ist.

3. Verfahren gemäß mindestens einem der Ansprüche 1 oder 2, **dadurch gekennzeichnet, dass** durch den Wärmetauscher (3) die zweite Reaktionszone (5) gekühlt wird.

4. Verfahren gemäß mindestens einem der Ansprüche 1 bis 3, **dadurch gekennzeichnet, dass** der Abstand zwischen der Bodenplatte (2) und dem Wärmetauscher (3) mindestens 30 mm beträgt.

5. Verfahren gemäß mindestens einem der Ansprüche 1 bis 4, **dadurch gekennzeichnet, dass** der Abstand zwischen der Bodenplatte (2) und dem Wärmetauscher (3) maximal 600 mm beträgt.

6. Verfahren gemäß mindestens einem der Ansprüche 1 bis 5, **dadurch gekennzeichnet, dass** der Wärmetauscher (3) wenigstens ein Kühlschlangenbündel umfasst.

7. Verfahren gemäß mindestens einem der Ansprüche 1 bis 6, **dadurch gekennzeichnet, dass** der heterogene Katalysator als Wirkkomponente Kupfer enthält.

8. Verfahren gemäß mindestens einem der Ansprüche 1 bis 7, **dadurch gekennzeichnet, dass** der Katalysator teilchenförmig ist und vorzugsweise einen mittleren Komdurchmesser von 40 µm bis 80 µm aufweist.

9. Wirbelschichtreaktor (1) zur Herstellung eines halogenierten Kohlenwasserstoffs umfassend mindestens einen Einlass für den Eduktstrom, mindestens einen Auslass für den Produktstrom, eine über dem Einlass angeordnete gasdurchlässige Bodenplatte (2) und einen oberhalb der Bodenplatte (2) gebildeten Reaktionsraum, wobei in dem Reaktionsraum wenigstens ein Wärmetauscher (3) angeordnet ist, der einen solchen Abstand von der Bodenplatte (2) aufweist, dass sich eine erste Reaktionszone (4) und eine zweite Reaktionszone (5) bilden, wobei die erste Reaktionszone (4) nicht durch den Wärmetauscher (3) temperiert wird.

10. Wirbelschichtreaktor (1) gemäß Anspruch 9, **dadurch gekennzeichnet, dass** der Wärmetauscher (3) in der zweiten Reaktionszone (5) so ausgelegt ist, dass er gekühlt werden kann.

11. Wirbelschichtreaktor (1) gemäß Anspruch 9 oder 10, **dadurch gekennzeichnet, dass** der Abstand zwischen der Bodenplatte (2) und dem Wärmetauscher (3) mindestens 30 mm beträgt.

12. Wirbelschichtreaktor (1) gemäß mindestens einem der Ansprüche 9 bis 11, **dadurch gekennzeichnet, dass** der Abstand zwischen der Bodenplatte (2) und dem Wärmetauscher (3) maximal 600 mm beträgt.

13. Wirbelschichtreaktor (1) gemäß mindestens einem der Ansprüche 9 bis 12, **dadurch gekennzeichnet, dass** der Wärmetauscher (3) wenigstens ein Kühlschlangenbündel umfasst.
